# EUROPEAN PATENT SPECIFICATION

(11) **EP 1 850 756 B1**
(45) Date of publication and mention of the grant of the patent: **21.01.2015**
(21) Application number: 06734752.6
(22) Date of filing: 08.02.2006
(51) Int. Cl.: A61B 6/04, G01N 21/00, A61N 5/10

(54) **A RIGID PATIENT SUPPORT ELEMENT FOR LOW PATIENT SKIN DAMAGE WHEN USED IN A RADIATION THERAPY ENVIRONMENT**
STEIFES PATIENTENSTÜTZELEMENT ZUR REDUKTION VON HAUTSCHÄDEN IN EINER STRAHLENTHERAPIE-UMGEBUNG
ELEMENT DE SUPPORT RIGIDE POUR PATIENT ENGENDRANT DE FAIBLES DEGATS CUTANES, LORS DE SON UTILISATION DANS UN ENVIRONNEMENT DE RADIOTHERAPIE

(30) Priority: 08.02.2005 US 650859 P; 18.05.2005 US 682321 P
(43) Date of publication of application: 07.11.2007
(73) Proprietor: QFix Systems LLC, Avondale, PA 19311 (US); Coppens, Daniel, Avondale, PA 19311 (US); Kirk, John Damon, Ramsey, NJ 07446 (US); Rabeno, David, Bear, DE 19701 (US); Winward, Thomas, Newark, DE 19702 (US)
(72) Inventor: COPPENS, Daniel, Avondale, Pennsylvania 19311 (US); KIRK, John Damon, Ramsey, New Jersey 07446 (US); RABENO, David, Bear, Delaware 19701 (US); WINWARD, Thomas, Newark, Delaware 19702 (US)
(74) Representative: Buckley, Guy Julian
(86) International application number: PCT/US2006/004764
(87) International publication number: WO 2006/086650

(56) References cited:
- WO-A-94/08542
- WO-A2-03/005881
- GB-A- 1 435 223
- GB-A- 2 320 685
- US-A- 3 947 686
- US-A- 4 145 612
- US-A- 4 312 912
- US-A- 5 537 454
- US-A- 5 771 513

## Description

### BACKGROUND OF THE INVENTION

Patients are commonly exposed to x-radiation from 1 MV to 50 MV during modern medical treatment and diagnostic procedures. X-rays are electromagnetic waves composed of photons. During treatment, a patient is often lying on a rigid support surface that is composed of carbon composite materials. When an x-radiation photon strikes an electron contained in the support surface, the photon scatters in one direction while the electron scatters in another direction. The effect is similar to the collision of two billiard balls on a pool table and is known as Compton scattering. It is these scattered electrons that strike a patient during treatment with x-radiation resulting in high skin dose and damage to the patient's skin.

Traditional patient support devices used in radiation therapy often use an open cross-weave of polymer monofilaments strung taught in the same manner as a tennis racquet as disclosed in US-A-5537454. This generally performs well from the standpoint of patient surface dosage but is impractical for many patient support surfaces and devices. In addition, it does not provide the precise positioning required for state of the art treatment techniques such as Inter-Modulated Radiation Therapy (IMRT) and Image Guided Radiation Therapy (IGRT).

Diagnostic imaging table technology has been used successfully in radiation therapy to a certain extent. However, the dosage that occurs at the contact point between the patient and support surface can be high. Diagnostic imaging tables are generally manufactured from continuous solid carbon fiber skins on a foam core as disclosed in US-A-3947686. Skin burn occurs because the carbon fiber layer results in electron generation by Compton scattering. Some of this electron energy is directed at the patient and because electrons travel a relatively short distance, a large dose of electron energy may be deposited in the patients skin, causing serious skin damage. Therefore, there is a need for a rigid patient support element that can reduce or eliminate radiation skin damage yet provide precise positioning of a patient.

In addition, modern radiation therapy positioning systems require structures that are rigid and substantially transparent to high-energy radiation. Current solutions are either rigid but produce an undesirable amount of electrons or are not adequately rigid but reduce electron generation. The present invention relates to rigid patient support elements for low patient skin damage when used in a radiation therapy environment. Document GB 2320685 discloses a patient support element according to the preamble of claim 1.

### SUMMARY OF THE INVENTION

The present invention, in its various aspects, is as set out in the accompanying claims.

The present invention provides a significant improvement to the state-of-the-art and solves the aforementioned problems because it is highly rigid while significantly decreasing the patient radiation skin dosage caused by electron scatter. The invention works seamlessly with newer equipment which is becoming available on the market such as cone-beam CT. In addition, in preferred embodiments, this invention is MRI compatible as well. The material is also well suited to be used in proton and other high-energy radiation therapy environments.

Specifically, the present invention provides a rigid patient support element that is substantially transparent to high energy x-radiation comprising a low density substantially x-ray transparent structural core with a top side and a bottom side, wherein the structural core comprises at least 50% open area and wherein the open structural core is at least one selected from the group consisting of columns and tubes; and one or more perforated face sheets attached to at least one of the- top side or bottom side, wherein the one or more of said face sheets comprise more than 50% open area. The support element reduces Compton scattering when exposed to x-radiation from 1 MV to 50 MV. The core comprises a low x-ray attenuating structural foam or a substantially open structure.

The present invention also provides a radiation therapy device, insert or spacer comprising the patient support element of the present invention.

The invention may be used in a method for reducing skin burn comprising the steps of providing a patient support surface; placing a rigid patient support element of the present invention on the patient support surface; placing a patient on the rigid patient support element; directing a high energy radiation beam through the patient support surface and the rigid patient support element into the patient; and reducing skin damage by diffusing the electrons generated by Compton scattering in the patient support surface.

### BRIEF DESCRIPTION OF THE DRAWINGS

FIG. 1 is a typical construction of the present invention showing face sheet material oriented in the 0°, 90°, and +/- 45° directions.
FIG. 2 illustrates a typical layered construction of the present invention.
FIG. 3 shows data comparing the dose vs. depth performance of several support elements of the present invention compared to air and a conventional patient support device.
FIG. 4 shows a detailed view of an optimized face sheet construction having material oriented in the 0°, 90°, and +/- 45° directions.
FIG. 5 is a typical patient table component made from a rigid support element of the present invention
FIG. 6 shows a view of an optimized face sheet construction having material oriented in the 0° and 90° directions.
FIG. 7 shows a view of an optimized face sheet construction having material oriented in the 0° and 90° directions wherein more material lies in the 0° direction than in the 90° direction.
FIG. 8 is a typical rigid patient support element of the present invention on which a patient is placed.
FIG. 9 is a typical rigid patient support element of the present invention placed on a conventional patient support element.
FIG. 10 is a traditional radiation therapy head & neck immobilization device which may be improved by the incorporation of the present invention.
FIG. 11 is a traditional radiation therapy patient treatment table which can be improved by the incorporation of the present invention.
FIG. 12 is a typical radiation therapy breast cancer treatment device which can be improved by the incorporation of the present invention.
FIG. 13 illustrates a square section of the present invention with round holes comprising 73% open area.
FIG. 14 illustrates a square section of the present invention with face sheets optimized for loads such as torsion and bending.

### DETAILED DESCRIPTION OF THE INVENTION

Currently, the two most common constructions for Radiation Therapy (RT) patient tables and devices are; (1) continuous solid carbon fiber face sheets on a rigid foam core, and (2) a carbon fiber grid. The grid is generally produced from a flat sheet of carbon fiber (between 0.1 and 0.635 cm (0.040" and 0.250")) through which a repeating pattern of square or round holes is cut. The grid provides superior performance when compared to a rigid foam core construction because it produces less electron scattering. However, the grid systems are much less stiff than the rigid foam systems.

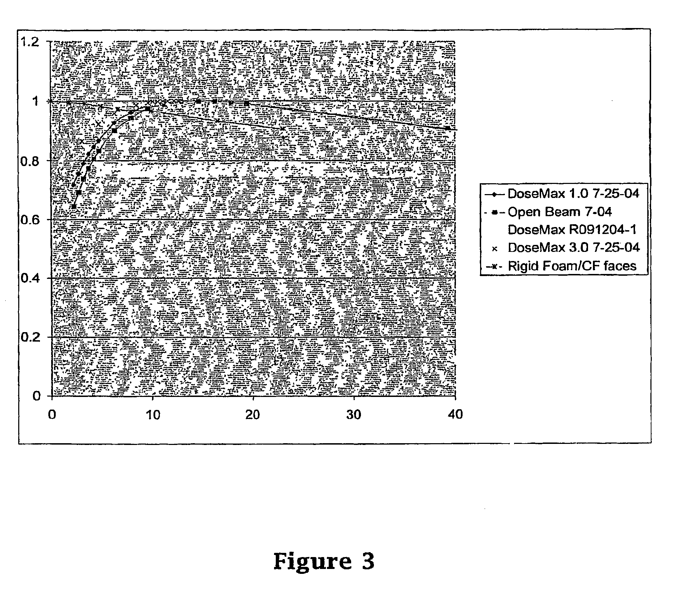

The invention described allows structures with electron generation comparable to a grid to be built but at an order of magnitude higher stiffness than a grid system. The invention can provide stiffness comparable to a rigid foam structure. Figure 3 illustrates the dose versus depth curve for a one inch and three inch sample of the present invention compared with a sample with structural integrity of a complete patient table, an open beam (with no structure between the patient and the x-ray source) as well as a stock rigid foam patient table. The present invention performs unexpectedly well compared with presently available solutions.

In addition to the reduction of electron energy affecting the patient's skin, rigidity has become a much more important parameter with the advent of IMRT and other high accuracy radiation therapy treatment methods. As the beam size becomes smaller and the tumor is targeted with pinpoint accuracy, it has become extremely important to know exactly where the patient is in the beam and to ensure that the patient does not move with respect to the beam. For this reason, a rigid surface is desirable. In addition, a rigid surface that does not produce scattered electrons is very desirable. The present invention provides a solution to these challenges.

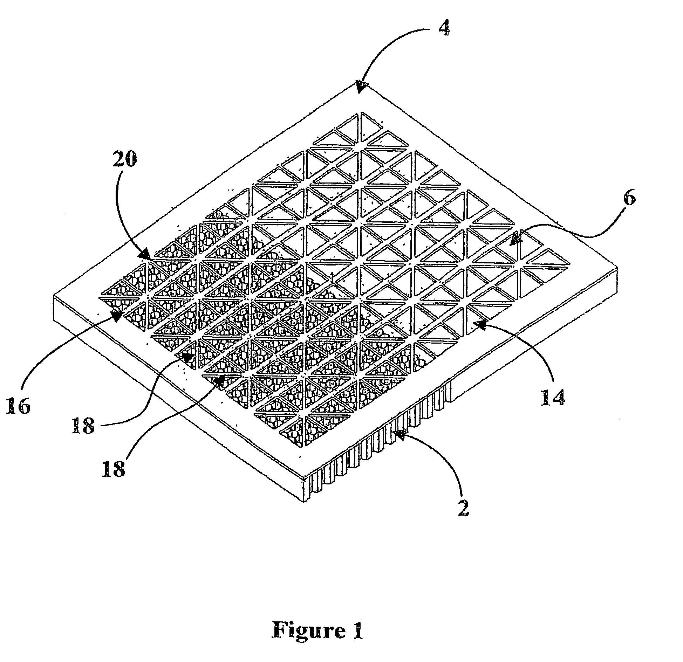

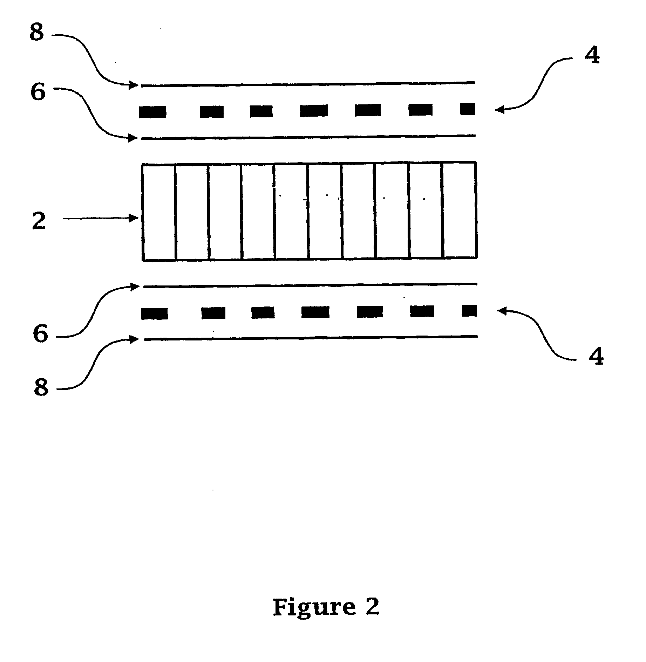

Referring to Figures 1 and 2, a low x-ray generating rigid structure is produced using a low density substantially x-ray transparent structural core material 2. The core material 2 is at least one selected from the group consisting of aramid, cellulose, carbon fiber, paper, polymer and phenolic. The structural core 2 can consist of thin walled substantially vertical tubes or columns 2 (as shown in Figure 1) on to which is placed at least one perforated face sheet 4. In a preferred embodiment, aramid honeycomb core is used as this material is readily available and has been shown to perform well for this purpose. However, circular, triangular, square or any other tubular shape, including three, four, five and six sided structures, will work as a core material, as long as the core is substantially open (greater than 50%). In addition, vertical columns such as carbon fiber rod can be used which, when installed in alternating angles from vertical, forms a truss structure, thereby increasing shear strength and stiffness.

Face sheets 4 can be placed on one or more sides of the structural core 2 and can be manufactured from a variety of suitable low x-ray absorbing materials including plastic and composite sheet. Specifically, the one or more face sheets may comprises at least one selected from the group consisting of carbon fiber, aramid fiber, man made fiber, cotton, wood pulp, natural fiber, UHMW Poly Ethylene, PBT, polymer, PPO, PS, PEEK, PEI, ABS, polyester, poly carbonate, acrylic, acrylonitril-butadiene-styrene, polyphenylene oxide, polyethylene, polypropylene, polyester, polyether ether ketone, polyetherimide, polyolefin and phenolic, liquid crystal polymer.

Composite materials such as carbon fiber, aramid fiber and PBO are all satisfactory material as long as the surface is substantially open (greater that 50% open area). The thickness of the face sheet can range from about 0.0254cm (0.010") up to as much as 0.952cm (0.375"). The face sheets 4 can be attached to the core 2 with one or more thin films of adhesive 6, such that the electron generation properties are minimally affected. This adhesive layer 6 can be perforated or solid. By using a thin solid layer, the core 2 is sealed (or closed out), protecting it from accumulating dirt and other contaminants. In addition, the adhesive layer 6 can serve to stabilize the core material 2, increasing its apparent strength and rigidity. In a preferred embodiment, the adhesive layer is reinforced with a composite material such as a carbon fiber or aramid fabric. Since these fabrics are readily available in thickness around 0.0254cm (0.010"), this is a very practical and useful construction. Because MRI compatible materials such as aramid are options for each portion of the construction, an MRJ compatible embodiment of this invention is readily achieved. For example, Kevlar® face sheets may be used on a Nomex® honeycomb core.

Optionally, as show in Figure 2, the grid face sheets 4 can also be covered with a thin perforation-free layer 8 to prevent contaminants from entering the structure and does not substantially affect Compton scattering. This is highly desirable in medical environments where cleanliness is extremely important. This surface sealing layer 8 can be produced from a thin polymer or composite sheet. We have found that a 0.0127 to 0.0254 cm (0.005" to 0.010") thick polycarbonate film produces a highly durable outer surface. Polyester film also works adequately. When a transparent layer is used, it has the added aesthetic value of allowing the end user to see the underlying structure.

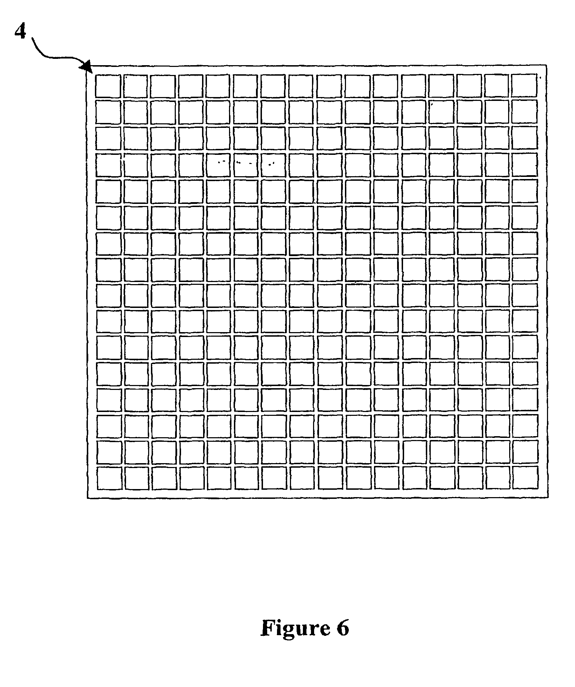

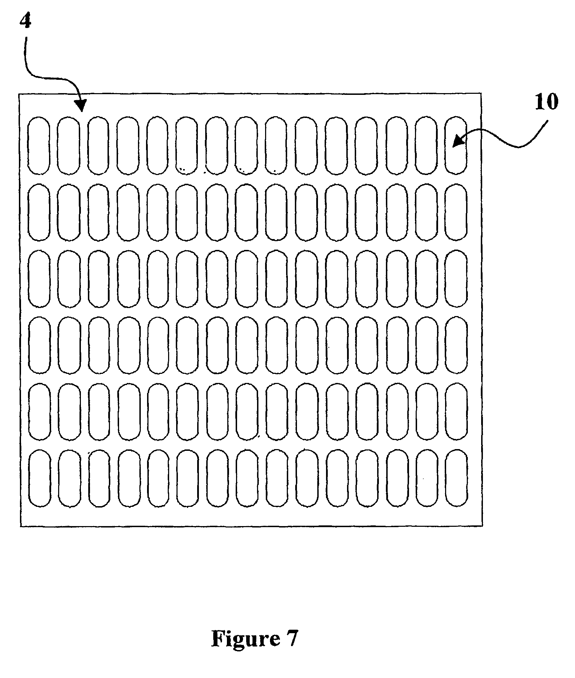

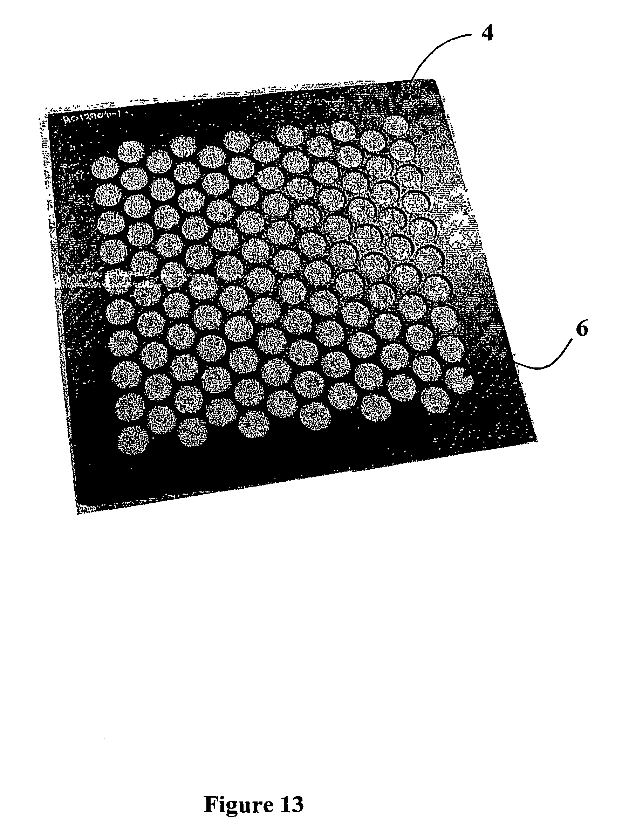

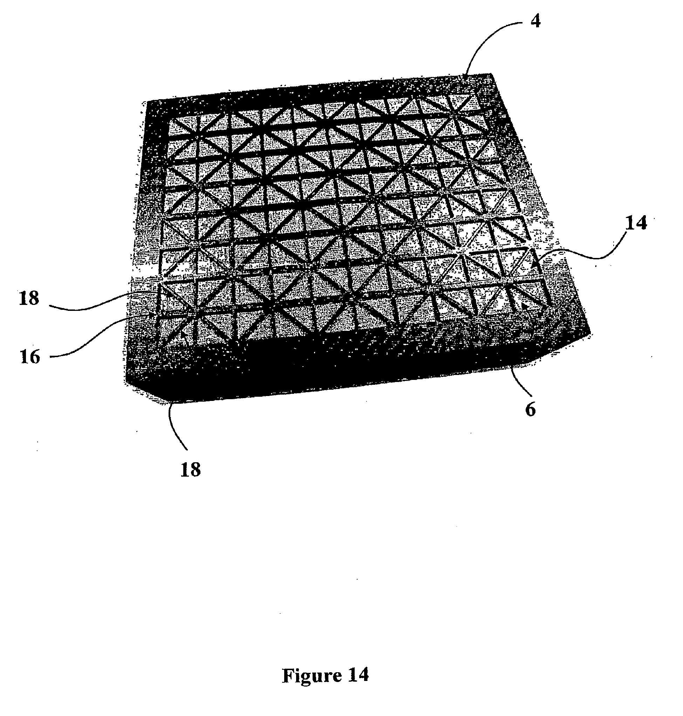

A beneficial aspect of this invention is that the grid pattern in the face sheets can be optimized to provide the maximum structural benefit to the system while maintaining minimum electron generation. Figures 6 and 7 show optional grid patterns while Figures 13 and 14 illustrate the use of grid patterns to achieve a certain open area or structural integrity. For example, one can create abeam simply supported at either end (as would be required for a Varian Exact® treatment table insert). As shown in Figure 7, one can produce oblong holes in the face sheets 10, leaving most of the composite spanning the length of the beam. In this way we have optimized on bending strength and stiffness in the direction of structural loads, shown here as 0°, while maintaining a high level of open area.

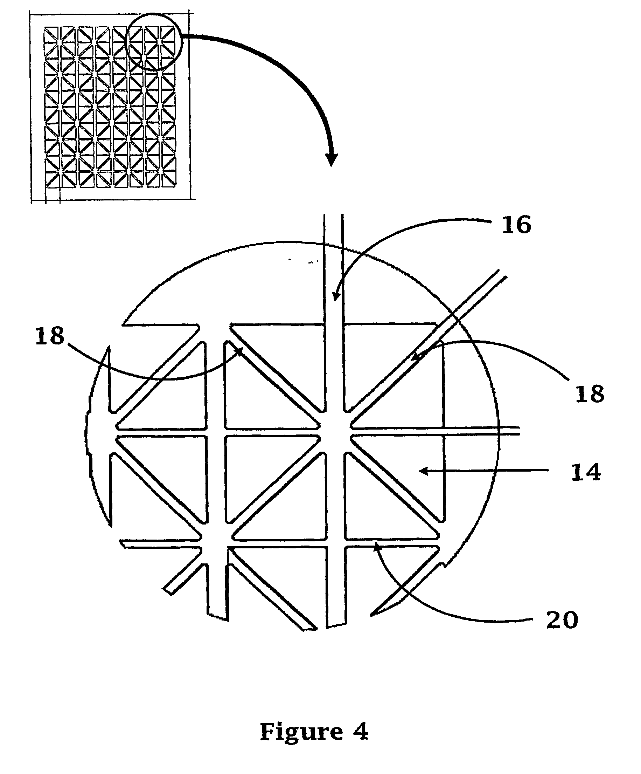

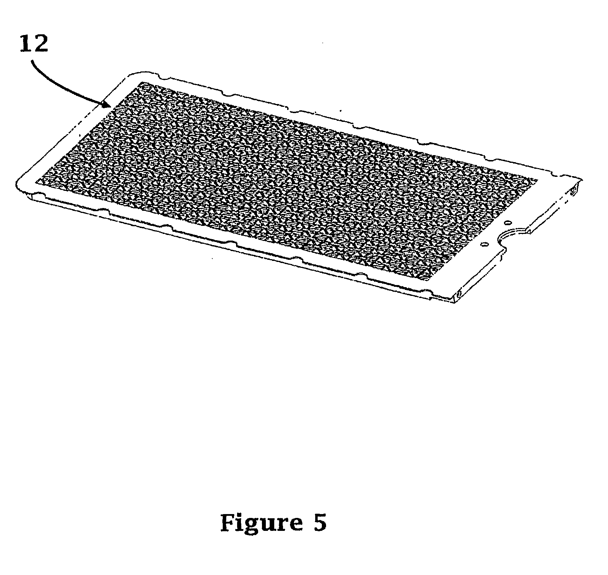

Another example and application of the present invention is shown in Figures 4 and 5 which is a patient table support section 12 created from the present invention. In this case, the table experiences both bending and torsional loadings. By using triangular openings 14 in the face sheets 4 and spacing them accordingly, one can optimize the amount of material in the 0 degree 16 and +/- 45 degree 18 directions. Figures 4 and 5 also illustrate the use of continuous material in the 90 degree 20 direction as well since this is generally of use in maintaining structural integrity. Of course, based on design requirements, any perforation configuration can be used to allow orientation of the face sheet material in the direction of structural loads.

Selection of the spacing and perforation pattern of the face sheets can be made depending on the desired characteristics. For example, it is not necessary to align the perforation pattern or to use the same perforation pattern on both sides of the structural core. It can, in fact, be desirable to do otherwise to optimize the structure and to minimize electron generation. For example, if a rigid support element of this invention is used in bending, one face sheet will be placed in tension while the other is placed in compression. Since most fiber systems display less stiffness and strength in compression than in tension, one can make the compression side face sheet ligaments wider and/or thicker.

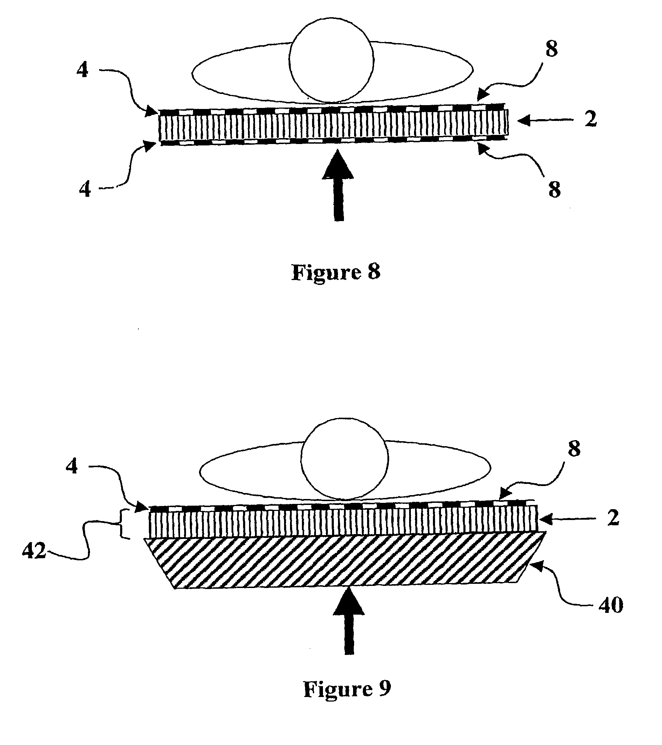

Figure 8 illustrates a typical use of the present invention. Radiation (represented by the arrow) passes though the rigid patient support element before entering the patient. Because the rigid patient support element reduces Compton scattering, patient skin damage is reduced.

Figure 9 shows another use of this material by spacing a patient away from a conventionally constructed table top or device **40.** Radiation (represented by the arrow) passes through table top or device **40.** The electrons generated by Compton scattering in the underlying support element can then be diffused before reaching the patient. By placing a section of the material of the present invention between the patient and device **40,** electrons generated in the device can be diffused before they enter the patient's skin. This reduces the local electron dose thereby minimizing skin damage. Since the spacer **42** provides a rigid patient surface, spatial positioning accuracy is maintained, allowing the radiation beam to be aimed properly. In this application, the bottom face sheet **4** can have a greater amount of open space or optionally be eliminated entirely, leaving the bottom open or simply covering it with a thin film.

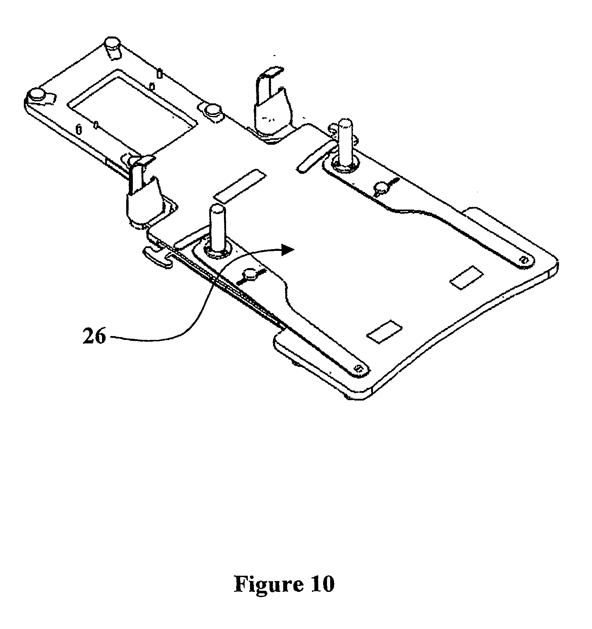

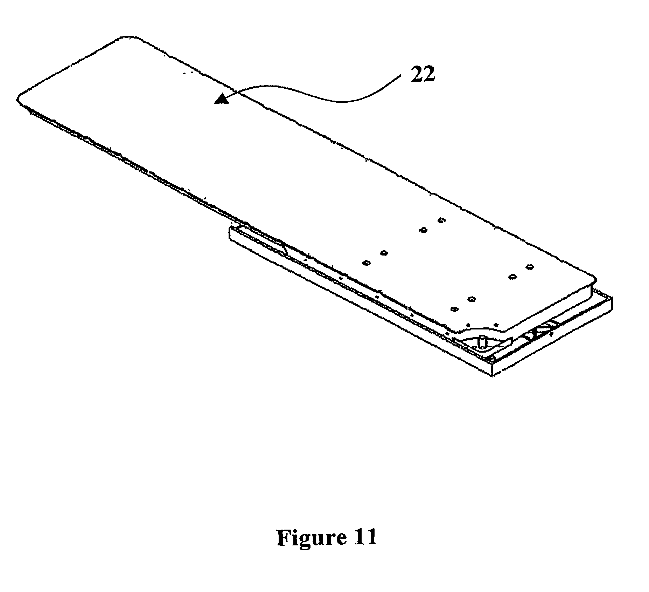

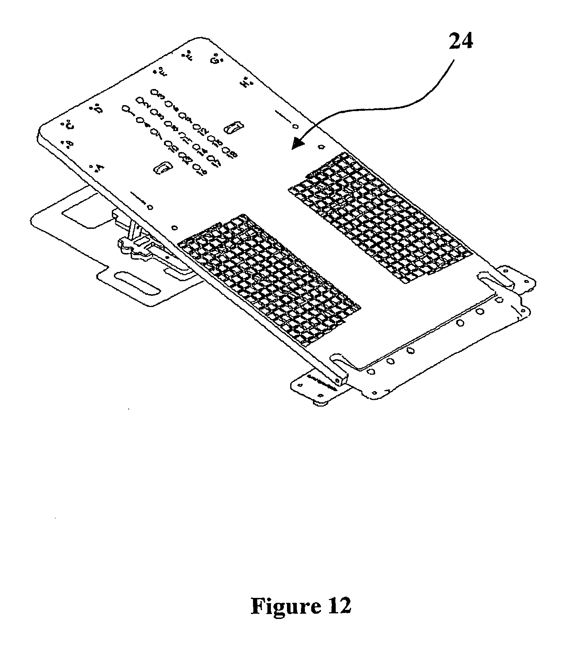

The present invention can be used and is compatible with conventional patient positioning devices and patient support tables. For example, a breast board, pelvis board or head and neck board, can incorporate the present invention thereby reducing or eliminating radiation skin burn at the specific treatment sites. Figures 10-12 illustrate the use of the present invention in a variety of optimized devices for radiation therapy including but not limited to patient tables **22,** devices, breast boards **24,** head & neck treatment boards **26,** lung treatment devices and pelvis boards.

The instant invention also provides a method for reducing skin damage comprising the steps of providing a patient support surface; placing a rigid patient support element of the present invention on the patient support surface; placing a patient on the rigid patient support element; directing a high energy radiation beam through the patient support surface and the rigid patient support element into the patient; and diffusing the electrons generated by Compton scattering in the patient support surface thereby reducing skin damage. Alternatively, the instant invention provides a method of reducing skin damage where the patient support surface comprises the rigid element of the present invention.

The present invention is further defined by the following claims.

## Claims

1. A rigid patient support element, that is substantially transparent to high energy x- radiation and reduces Compton scattering when exposed to x-radiation from 1 MV to 50 MV, comprising:
a) a low density substantially x-ray transparent structural core (2) with a top side and bottom side; and
b) one or more perforated face sheets (4) attached to at least one of the top side or bottom side, wherein the one or more of said face sheets comprise more than 50% open area;
wherein the structural core comprises at least 50% open area and **characterized in that** the open structure core is at least one selected from the group consisting of columns and tubes.

2. The rigid patient support element of claim 1 wherein the columns or tubes have at least one cross-section selected from the group consisting of; circle (forming cylinders), three sided, four sided, five sided, six sided, triangle, square, rectangle, rhombus, pentagon, and hexagon (forming a honeycomb structure).

3. The rigid patient support element of claim 1 wherein the open structure core material is at least one selected from the group consisting of aramid, cellulose, carbon fiber, paper, polymer and phenolic.

4. The rigid patient support element of claim 1 wherein the one or more perforated face sheet comprises at least one selected from the group consisting of; carbon fiber, aramid fiber, man made fiber, cotton, wood pulp, natural fiber, UHMW Poly Ethylene, PBT, polymer, PPO, PS, PEEK, PEI, ABS, polyester, poly carbonate, acrylic, acrylonitril-butadiene-styrene, polyphenylene oxide, polyethylene, polypropylene, polyester, polyether ether ketone, polyetherimide, polyolefin and phenolic, liquid crystal polymer.

5. The rigid patient support element of claim 1 wherein the one or more perforated face sheets are attached to the substantially open structure core by one or more adhesive layers.

6. The rigid patient support element of claim 5 wherein the adhesive layer is perforated.

7. The rigid patient support element of claim 5 wherein the adhesive layer is perforation free.

8. The rigid patient support element of claim 5 wherein the adhesive layer is fiber reinforced.

9. The rigid patient support element of claim 1 on to which is adhered a thin perforation free outer surface layer wherein said outer surface layer does not substantially affect the Compton scattering.

10. The rigid patient support element of claim 1 that is compatible with Magnetic Resonance Imaging.

11. The rigid patient support element of claim 1 wherein the perforation of the one or more perforated face sheets are tailored to enhance structural performance by allowing the face sheet material to be oriented in the direction of structural loads.

12. Use of the rigid patient support element of claim 1 in a radiation therapy device, as a radiation therapy patient table insert or as a radiation therapy spacer.

## Patentansprüche

1. Ein steifes Patientenstützelement, das im Wesentlichen transparent für hochenergetische Röntgenstrahlung ist und die Compton-Streuung reduziert, wenn es einer Röntgenstrahlung von 1 MV bis 50 MV ausgesetzt ist, umfassend:
a) einen Strukturkern (2) mit geringer Dichte und im Wesentlichen transparent für Röntgenstrahlung, mit einer Oberseite und Unterseite; und
b) eine oder mehrere perforierte Deckschichten (4) auf mindestens einer der Oberseite oder der Unterseite befestigt, wobei die eine oder mehrere der besagten Deckschichten mehr als 50% Durchlässigkeit aufweisen,
wobei der Strukturkern mindestens 50% Durchlässigkeit aufweist, **dadurch gekennzeichnet, dass** der offene Strukturkern mindesten eines ausgewählt aus der Gruppe bestehend aus Säulen und Röhren ist.

2. Das steife Patientenstützelement gemäß Anspruch 1, wobei die Säulen oder Röhren mindestens einen Querschnitt haben, ausgewählt aus der Gruppe bestehend aus kreisförmig (bildet Zylinder), dreiseitig, vierseitig, fünfseitig, sechsseitig, dreieckig, quadratisch, rechteckig, rhombisch, pentagonal, hexagonal (bildet Wabenstruktur).

3. Das steife Patientenstützelement gemäß Anspruch 1, wobei das Material des offenen Strukturkerns mindestens eines ausgewählt aus der Gruppe bestehend aus Aramid, Cellulose, Kohlenstoff-Fasern, Papier, Polymeren und Phenol ist.

4. Das steife Patientenstützelement gemäß Anspruch 1, wobei mindestens eine der einen oder mehreren perforierten Deckschichten ausgewählt ist aus der Gruppe bestehend aus Kohlenstofffaser, Aramidfaser, Kunstfaser, Baumwolle, Holzzellstoff, Naturfaser, UHMW Polyethylen, PBT, Polymer, PPO, PS, PEEK, PEI, ABS, Polyester, Polycarbonat, Acryl, Acrylnitril-Butadien-Styrol, Polyphenylenoxid, Polyethylen, Polypropylen, Polyester, Polyether-Etherketon, Polyetherimid, Polyolefin und Phenolharz, Flüssigkristallpolymer.

5. Das steife Patientenstützelement gemäß Anspruch 1, wobei die eine oder mehreren perforierten Deckschichten an dem im Wesentlichen offen Strukturkern durch eine oder mehrere Klebeschichten befestigt sind.

6. Das steife Patientenstützelement gemäß Anspruch 5, wobei die Klebeschicht perforiert ist.

7. Das steife Patientenstützelement gemäß Anspruch 5, wobei die Klebeschicht ohne Perforation ist.

8. Das steife Patientenstützelement gemäß Anspruch 5, wobei die Klebeschicht faserverstärkt ist.

9. Das steife Patientenstützelement gemäß Anspruch 1 auf welches eine dünnere äußere Oberflächenschicht ohne Perforation geklebt ist, wobei die besagte äußere Oberflächenschicht die Compton-Streuung im Wesentlichen nicht beeinflusst.

10. Das steife Patientenstützelement gemäß Anspruch 1 welches kompatibel mit Kernspintomographie ist.

11. Das steife Patientenstützelement gemäß Anspruch 1, wobei die Perforation der einen oder mehreren perforierten Deckschichten derart zugeschnitten ist, um die Strukturleistung zu erhöhen, indem das Deckschichtmaterial in Richtung der Strukturlasten orientiert ist.

12. Die Verwendung von dem steifen Patientenstützelement gemäß Anspruch 1 in einem Gerät zur Strahlentherapie, als eine Tischeinlage für Patienten einer Strahlentherapie, oder als Distanzstück.

## Revendications

1. Elément support rigide pour patients qui est essentiellement transparent aux rayons x de haute énergie et réduit l'effet Compton, lorsqu'il est exposé aux rayons X de 1 meV à 50 meV, comprenant :
a) un noyau structurel (2) à faible densité essentiellement transparent aux rayons X avec un côté supérieur et un côté inférieur,
b) une ou plusieurs feuilles de couverture perforées (4) fixées à l'au moins un parmi le côté supérieur ou le côté inférieur, lesdites une ou plusieurs feuilles de couverture comprenant plus de 50% de zone ouverte,
le noyau structurel comprenant au moins 50% de zone ouverte, et **caractérisé en ce que** le noyau de la structure ouverte est au moins un sélectionné parmi le groupe constitué de colonnes et de tubes.

2. Elément support rigide pour patients suivant la revendication 1, dans lequel les colonnes ou tubes ont au moins une section transversale sélectionnée parmi le groupe constitué de cercles (formant des cylindres), de formes à trois côtés, à quatre côtés, à cinq côtés, à six côtés, triangulaire, carrée, rectangulaire, rhombique, pentagonale et hexagonale (formant une structure en nid d'abeille).

3. Elément support rigide pour patients suivant la revendication 1, dans lequel la matière du noyau de la structure ouverte est au moins une sélectionnée parmi le groupe constitué d'aramide, de cellulose, de fibre de carbone, de papier, de polymère et phénolique.

4. Elément support rigide pour patients suivant la revendication 1, dans lequel l'une ou les plusieurs feuilles de couverture perforées comprennent au moins un composant sélectionné parmi le groupe constitué de fibres de carbone, de fibres d'aramide, de fibres artificielles, de coton, de pâte de bois, de fibres naturelles, de polyéthylène UHMW, PBT, polymère, PPO, PS, PEEK, PEI, ABS, polyester, polycarbonate, acrylique, acrylonitrile-butadiène-styrène, oxyde de polyphénylène, polyéthylène, polypropylène, polyester, polyétheréthercétone, polyétherimide, polyoléfine et phénolique, polymère à cristaux liquides.

5. Elément support rigide pour patients suivant la revendication 1, dans lequel l'une ou les plusieurs feuilles de couverture perforées sont fixées au noyau de la structure essentiellement ouverte au moyen d'une ou plusieurs couches adhésives.

6. Elément support rigide pour patients suivant la revendication 5, dans lequel la couche adhésive est perforée.

7. Elément support rigide pour patients suivant la revendication 5, dans lequel la couche adhésive est exempte de perforations.

8. Elément support rigide pour patients suivant la revendication 5, dans lequel la couche adhésive est renforcée avec des fibres.

9. Elément support rigide pour patients suivant la revendication 1, sur lequel est collée une mince couche de couverture extérieure exempte de perforations, ladite couche de couverture extérieure n'affectant pas essentiellement l'effet de Compton.

10. Elément support rigide pour patients suivant la revendication 1, qui est compatible avec l'imagerie par résonance magnétique.

11. Elément support rigide pour patients suivant la revendication 1, dans lequel la perforation de l'une ou des plusieurs feuilles de couverture perforées est adaptée pour améliorer la performance structurelle en permettant à la feuille de couverture d'être orientée dans la direction de charges structurelles.

12. Utilisation de l'élément support rigide pour patients suivant la revendication 1 dans un dispositif de radiothérapie en tant qu'insert dans une table de traitement par radiothérapie ou en tant que dispositif espaceur en radiothérapie.
